# EUROPÄISCHE PATENTSCHRIFT

(11) **EP 0 592 476 B1**
(45) Veröffentlichungstag und Bekanntmachung des Hinweises auf die Patenterteilung: **30.10.1996**
(21) Anmeldenummer: 92912592.0
(22) Anmeldetag: 29.06.1992
(51) Int. Cl.: A61K 38/00, A61K 48/00, C12N 15/24

(54) **VERWENDUNG VON INTERLEUKIN 10 ZUR HERSTELLUNG VON ARZNEIMITTELN MIT TUMORHEMMENDER WIRKSAMKEIT**
USE OF INTERLEUKINE 10 TO PRODUCE DRUGS WITH A TUMOUR-INHIBITING ACTION
UTILISATION D'INTERLEUKINE 10 POUR LA FABRICATION DE MEDICAMENTS A EFFET D'INHIBITION DES TUMEURS

(30) Priorität: 04.07.1991 DE 4122402
(43) Veröffentlichungstag der Anmeldung: 20.04.1994
(73) Patentinhaber: SCHERING AKTIENGESELLSCHAFT, 13342 Berlin (DE)
(72) Erfinder: DIAMANTSTEIN, Tibor, D-1000 Berlin 19 (DE); RICHTER, Günther, D-1000 Berlin 61 (DE); BLANKENSTEIN, Thomas, D-1000 Berlin 45 (DE)
(86) Internationale Anmeldenummer: EP9201405
(87) Internationale Veröffentlichungsnummer: WO9300920

(56) Entgegenhaltungen:
- EP-A- 0 405 980
- WO-A-92/12725
- CELL. Bd. 60, 9. Februar 1990, CAMBRIDGE, NA US Seiten 397 - 403; FEARON,E.R. ET AL: 'Interleukin-2 production by tumor cells bypasses T helper function in the generation of an antitumor response'
- IMMUNOLOGY TODAY Bd. 11, Nr. 6, Juni 1990, CAMBRIDGE U.K. Seiten 196 - 200; RUSELL, S.J.: 'Lymphokine gen therapy for cancer'
- JOURNAL OF IMMUNOLOGY. Bd. 145, Nr. 12, 15. Dezember 1990, BALTIMORE US Seiten 4167 - 4173; MACNEIL I.A. ET AL: 'IL-10, a novel growth cofactor for mature and immature T cells'

## Beschreibung

Die Erfindung betrifft die Verwendung von Interleukin 10 zur Herstellung von Arzneimitteln mit tumorhemmender Wirksamkeit.

Interleukine (IL) sind bekanntlich Mediator-Stoffe des Immunsystems, die vorwiegend von Leukozyten gebildet werden. Sie gehören zur Gruppe der sogenannten Cytokine; dies sind Stoffe, die von Zellen in das Blut oder umgebende Gewebe abgeschieden werden und die Funktionen anderer Zellen, wie zum Beispiel deren Teilung, Differenzierung oder Bewegung beeinflussen.

Die meisten Interleukine stimmulieren die Funktionen anderer Zellen. So ist beispielsweise das von den T1-Helferzellen ausgeschiedene Interleukin 2 (IL-2) befähigt, Makrophagen, natürliche Killerzellen, B-Lymphocyten und T-Lymphocyten - also die weißen Blutkörperchen, welche die Immunabwehr bewirken - zu aktivieren und ihre Vermehrung anzuregen (Kendall A. Smith: Science 240, 1988, 1169-1176 sowie Spektrum der Wissenschaft Mai 1990, 72-82).

Demgegenüber hemmt das in den T2-Helferzellen gebildete Interleukin 10 (IL-10) die Interleukin 2-Bildung und die γ- Interferon-Bildung in den T1-Helferzellen, weshalb man es auch als cytokine synthesis inhibitory factor (CSIF) bezeichnet und bewirkt demzufolge eine Schwächung des Immunsystems (David F. Fiorentino et. al.: J. Exp. Med. 170, 1989 2081-2095; Ning Fei Go et. al.: J. Exp. Med. 172, 1990, 1625-1631; Tim R. Mosmann et. al.: J. of Immunology 145, 1990 2938-2945; P. Vieira et. al.: Proc. Natl. Acad. Sci. 88, 1991, 1172-1176).

Es wurde nun gefunden, daß Interleukin 10 überraschenderweise eine tumorhemmende Wirksamkeit besitzt, wie dies im nachfolgenden Ausführungsbeispiel näher erläutert wird. Diese tumorhemmende Wirkung muß auf einer bislang unbekannten Eigenschaft des Interleukin 10 beruhen; dies ergibt sich bereits aus der Tatsache, daß Inteleukin 10 sowohl bei Mäusen, die über kein funktionsfähiges spezifisches Immunsystem verfügen (SCID-Mäuse, die werder T- noch B-Lymphozyten besitzen) als auch bei Mäusen, die über kein T-Zellabhängiges funktionsfähiges spezifisches Immunsystem besitzen (Nude Mäuse) eine tumorhemmende Wirksamkeit entfaltet.

Wegen dieser Eigenschaft kann Interleukin 10 zur Herstellung von Arzneimitteln mit tumorhemmender Wirksamkeit verwendet werden. Dies kann beispielsweise geschehen, indem man Interleukin 10 haltige Lösungen nach einem der Verfahren herstellt, die in den bereits erwähnten Publikationen von Fiorentino et. al., Ning Fei Go et. al., Mosmann et. al. oder Vieira et. al. beschrieben sind.

Im allgemeinen wird es ausreichend sein Lösungen bereitzustellen, die pro ml 10³ bis 10⁶ Einheiten Interleukin 10 enthalten. Diese Lösungen werden gegebenenfalls mit isotonisierenden Zusätzen versetzt, sterilfiltriert und in Ampullen oder Infusionsflaschen von 1 bis 250 ml Fassungsvermögen abgefüllt. Andererseits ist es aber auch möglich die Lösungen zu lyophilisieren um sie vor Gebrauch wieder aufzulösen.

Andererseits ist es aber auch möglich die IL-10 tragenden Plasmide, welche aus den bereits erwähnten Publikationen vorbekannt sind beispielsweise mittels Transfektion (E. Neumann et. al. EMBO Journal 1, 1982, 841-845) in Zellen, wie zum Beispiel TIL-Zellen (=tumor infiltrating leukocytes) einzubringen und die erhaltene Zellsuspension in Arzneimitteln zu verwenden.

Zur Behandlung benigner und maligner Tumore werden die erfindungsgemäßen Arzneimittel i.v. oder lokal appliziert. Die Menge des zu applizierenden Arzneimittels ist naturgemäß von der Art der Erkrankung und dem Zustand des Patienten abhängig und muß im Einzelfall vom Arzt festgelegt werden. Im allgemeinen wird es ausreichend sein, wenn man dem Patienten pro Tag 10² bis 10⁸ Einheiten Interleukin 10 verabreicht.

Das nachfolgende Ausführungsbeispiel dient zur näheren Erläuterung der Erfindung:

### BEISPIEL

a) Isolierung des Gens für das Maus IL-10.
   Das Maus IL-10 Gen wurde mit Hilfe von sequenzspezifischer Primer (sence:
   AGAGTCGACCATCATGCCTGGCTCAGCA und antisence:
   GCAGTCGATCTAGCTTTCCATTTTGATCATCA) durch "reverse transcription polymerase chain reaction (RT-PCR) (Ernst S. Kawaski et. al.: Proc. Natl. Acad. Sci., 85, 1988, 5698-5702) isoliert. Als RNA-Quelle dienten mit 3µg/ml Concanavalin A stimmulierte T-Zellen aus Mäuse-Milz. Mit Hilfe der oben genannten Primer wurden Restriktionsschnittstellen für Sal 1 in das Genamplifikationsprodukt eingeführt., das eine Klonierung in das entsprechend geschnittene Expressionsplasmid pBMGNeo (Hajime Karasuyama et. al.: Eur. J. Immunol. 18, 1988, 97-104) erlaubte.
b) Transfektion von CHO-Tumorzellen.
   5x10⁶ CHO-Tumorzellen (CHO = Chinese hamster ovary) Puck T.T; J. Exp. Med. 108, 1958, 945 wurden zweimal in Dulbeccos PBS ohne Ca²⁺ und Mg²⁺-Ionen gewaschen (Dulbecko, R.; J. Exp.. Med. 99, 1954, 167) und auf 0,5 ml eingestellt. Dann wurden sie in eine Küvette eines Elektroporators der Firma Biorad (Richmond, California-USA) gegeben mit 10µg linearisiertem IL-10-tragendem Plasmid vermischt und 10 Minuten bei 0 °C aufbewahrt. Anschließend erfolgte die Elektroporation bei 875 V/cm und r= 5 m sec (Bio-Radiations, Bio-Rad Laboratories 1981, 63-66).
   Die behandelten Zellen werden 10 Minuten lang bei 0 °C aufbewahrt, in 30 ml Click's RPMI-1640 Medium (Hersteller Biochrom GmbH; DE-Berlin) enthaltend 2 mM Glutamin, 100 Einheiten /ml Penicillin, 100µg/ml Streptomycin und 10 % föta les Kälberserum (Hersteller FCS/Seromed; DE-München) überführt und 48 Stunden lang bei 37 °C unter 5 % kohlendioxidhaltiger Atmosphäre kultiviert.
   Das auf dem IL-10 tragenden Expressionsplasmid enthaltende Neomycinresistenzgen diente als Selektionsmarker. Die Zellen wurden deshalb auf eine Konzentration von 1x10⁶/ml eingestellt mit 1 mg/ml des Neomycinanalogen G 418 (Hersteller GIBCO-BRL; DE-7514 Eggenstein) versetzt und 2 Wochen lang auf Antibiotikaresistenz selektioniert.
   Zur Bestimmung der biologischen Aktivität des IL-10 Gens wurden die Überstände der erhaltenen Klone in einem Proliferationsassay auf D-36 (Masterzellinie) getestet (Schmitt, E. et. al. Cytokine 2 (6), 1990, 407). Einer der Klone zeigte eine Aktivität von 256 Einheiten /ml auf 5x10⁴ Zellen in 24 Stunden. Diese Zellen wurden überwiegend für die weiteren Untersuchungen verwendet.
c) Bestimmungen der tumorhemmenden Wirkung von IL-10.
   Da die vom Hamster abstammenden CHO-Tumorzellen in Mäusen mit intaktem Immunsystem als Fremdtransplantate zerstört werden, wurden die Versuche zur Vermeidung der Xenotransplantatabstoßung mit SCID-Mäusen, die infolge ihrer T- und B-Lyphozyteninsuffiziens über kein funktionsfähiges spezifisches Immunsystem verfügen und mit Nude-Mäusen, die infolge ihrer T-Zelldefizienz über kein funktionsfähiges spezifisches Immunsystem verfügen, durchgeführt.
   10 SCID-Mäusen und 10 Nude-Mäusen wurden 5x10⁶ IL-10 bildende CHO-Tumorzellen, subcutan in den Nacken gespritzt. 25 Tage nach der Applikation lebten alle Mäuse. Tumore wurden nicht festgestellt.
   Als Kontrolle dienten 10 SCID-Mäuse und 10 Nude Mäuse, die 5x10⁶ CHO-Tumorzellen, welche zur IL-10 Bildung nicht befähigt sind subcutan in den Nacken appliziert bekamen. Die Untersuchung zeigte, daß sich bei allen Tieren nach 14 Tagen Tumore entwickelten. Alle Tiere waren spätestens nach 25 Tagen verstorben.

## Patentansprüche

1. Verwendung von Interleukin 10 zur Herstellung von Arzneimitteln mit tumorhemmender Wirksamkeit.

2. Verwendung von Interleukin 10 zur Herstellung von Arzneimitteln gemäß Patentanspruch 1, dadurch gekennzeichnet, daß das Arzneimittel eine Suspension von zur Interleukin 10 Bildung befähigten Zellen enthält.

## Claims

1. Use of interleukin 10 for the manufacture of medicaments having tumour-inhibiting activity.

2. Use of interleukin 10 for the manufacture of medicaments according to patent claim 1, characterised in that the medicament contains a suspension of cells capable of the formation of interleukin 10.

## Revendications

1. Utilisation de l'interleukine-10 pour la préparation de médicaments ayant une activité inhibitrice de tumeurs.

2. Utilisation de l'interleukine-10 pour la préparation de médicaments selon la revendication 1 caractérisée en ce que le médicament contient une suspension de cellules capables de former l'interleukine-10.
